# EUROPÄISCHE PATENTANMELDUNG

(11) **EP 4 834 196 A1**
(43) Veröffentlichungstag der Anmeldung: **04.11.2026**
(21) Anmeldenummer: 25173998.3
(22) Anmeldetag: 02.05.2025
(51) Int. Cl.: A61B 1/00, A61B 1/04

(54) **KUPPLUNGSVORRICHTUNG ZUM LÖSBAREN ANSCHLIESSEN EINES OKULARTRICHTERS EINES ENDOSKOPISCHEN SYSTEMS AN EINE KAMERA**

(71) Anmelder: Richard Wolf GmbH, 75438 Knittlingen (DE)
(72) Erfinder: Amann, Carina, 75449 Wurmberg (DE); Körner, Eberhard, 75438 Knittlingen (DE)
(74) Vertreter: Patentanwälte Hemmer Lindfeld Frese Partnerschaft mbB

(57) **Zusammenfassung**

Die vorliegende Erfindung betrifft eine Kupplungsvorrichtung (15) zum lösbaren Anschließen eines Okulartrichters (11) eines endoskopischen Systems (1) an eine Kamera (13), wobei die Kupplungsvorrichtung (15) an einer proximalen Seite (17) der Kupplungsvorrichtung (15) mit einer Kamera (13) verbunden oder lösbar verbindbar ist, wobei die Kupplungsvorrichtung (15) an einer in Richtung einer optische Achse (L) der proximalen Seite (17) der Kupplungsvorrichtung (15) gegenüberliegenden distalen Seite (19) der Kupplungsvorrichtung (15) mit einem Okulartrichter (11) lösbar verbindbar ist, wobei die Kupplungsvorrichtung (15) aufweist:
- eine an der distalen Seite (19) der Kupplungsvorrichtung (15) angeordnete Okulartrichteraufnahme (21), in welche ein Okulartrichter (11) eines endoskopischen Systems (1) manuell gedrückt werden kann, und
- mindestens zwei Verriegelungselemente (23) mit jeweils einem Verriegelungsabschnitt (25) zum Verriegeln eines Okulartrichters (11) in der Okulartrichteraufnahme (21), wobei die mindestens zwei Verriegelungselemente (23) jeweils zwischen einer Freigabeposition und einer Verriegelungsposition um eine dem jeweiligen Verriegelungselement (23) zugehörige Verriegelungsdrehachse (A) drehbar gelagert sind,
wobei der jeweilige Verriegelungsabschnitt (25) der mindestens zwei Verriegelungselemente (23) in der Verriegelungsposition des jeweiligen Verriegelungselements (23) in die Okulartrichteraufnahme (21) ragt und sich in der Freigabeposition des jeweiligen Verriegelungselements (23) radial außerhalb der Okulartrichteraufnahme (21) befindet, wobei die zu jedem der Verriegelungselemente (23) zugehörige Verriegelungsdrehachse (A) jeweils schräg zur optischen Achse (L) verläuft.

## Beschreibung

Die vorliegende Erfindung betrifft eine Kupplungsvorrichtung zum lösbaren Anschließen eines Okulartrichters eines endoskopischen Systems an eine Kamera.

Kupplungsvorrichtungen dieser Art dienen in der Regel dazu, ein ansonsten für die Betrachtung mit dem Auge dienendes Okular eines endoskopischen Systems an das Objektiv einer Kamera anzuschließen. Die EP 2 401 953 B1 beschreibt beispielsweise solch eine Kupplungsvorrichtung. Auch aus der DE 69 26 837 U ist eine derartige Kupplungsvorrichtung bekannt.

Eine wichtige Eigenschaft der Kupplungsvorrichtung soll sein, dass die Befestigung des Okulartrichters selbsttätig erfolgt. Das heißt, dass beim Einführen des Okulartrichters in eine entsprechend ausgestaltete Aufnahme der Kupplungsvorrichtung kein extra Entriegelungsschritt durchgeführt werden muss. In der EP 2 401 953 B1 ist dies beispielsweise durch ein automatisches Zuschnappen von Verriegelungshebeln realisiert.

Nachteilig an der aus der EP 2 401 953 B1 bekannten Lösung ist allerdings, dass die Verrieglungshebel nur relativ kurz ausgestaltet werden können und dadurch auf einen relativ engen Toleranzbereich beschränkt sind. Die Kupplungsvorrichtung ist dadurch wenig tolerant gegenüber einem Verkanten des Okulartrichters beim Einführen in die Kupplungsvorrichtung.

Dadurch ergibt sich als Aufgabe der vorliegenden Erfindung, eine Kupplungsvorrichtung bereitzustellen, die eine selbsttätige Befestigung eines Okulartrichters eines endoskopischen Systems an eine Kamera erlaubt, wobei die Kupplungsvorrichtung toleranter gegenüber Verkantungen, Verkippungen oder Fertigungstoleranzen seitens des Okulartrichters ist.

Gelöst wird diese Aufgabe durch eine Kupplungsvorrichtung gemäß Anspruch 1. Vorteilhafte Weiterbildungen und Ausführungsformen dieser Kupplungsvorrichtung ergeben sich aus den Unteransprüchen, der Beschreibung sowie den Zeichnungen. Hierbei können gemäß der Erfindung die in den Unteransprüchen oder der Beschreibung angegebenen Merkmale jeweils für sich, aber auch in technisch sinnvoller Kombination die erfindungsgemäße Lösung gemäß Anspruch 1 optional weiter ausgestalten.

Erfindungsgemäß wird eine Kupplungsvorrichtung bereitgestellt zum lösbaren Anschließen eines Okulartrichters eines endoskopischen Systems an eine Kamera, wobei die Kupplungsvorrichtung an einer proximalen Seite der Kupplungsvorrichtung mit einer Kamera verbunden oder lösbar verbindbar ist, wobei die Kupplungsvorrichtung an einer in Richtung einer optischen Achse der proximalen Seite der Kupplungsvorrichtung gegenüberliegenden distalen Seite der Kupplungsvorrichtung mit einem Okulartrichter lösbar verbindbar ist. Die Kupplungsvorrichtung weist erfindungsgemäß auf:
- eine an der distalen Seite der Kupplungsvorrichtung angeordnete Okulartrichteraufnahme, in welcher ein Okulartrichter eines endoskopischen Systems manuell gedrückt werden kann, und
- mindestens zwei Verriegelungselemente mit jeweils einem Verriegelungsabschnitt zum Verriegeln eines Okulartrichters in der Okulartrichteraufnahme, wobei die mindestens zwei Verriegelungselemente jeweils zwischen einer Freigabeposition und einer Verriegelungsposition um eine dem jeweiligen Verriegelungselement zugehörige Verriegelungsdrehachse drehbar gelagert sind.
Dabei ragt der jeweilige Verriegelungsabschnitt der mindestens zwei Verriegelungselemente in der Verriegelungsposition des jeweiligen Verriegelungselements in die Okulartrichteraufnahme und befindet sich in der Freigabeposition des jeweiligen Verriegelungselements radial außerhalb der Okulartrichteraufnahme, wobei die zu jedem der Verriegelungselemente zugehörige Verriegelungsdrehsachse jeweils schräg zur optischen Achse verläuft. "Schräg" soll hier bedeuten, dass die jeweilige Verriegelungsdrehachse weder parallel zur optischen Achse verläuft noch in einer Ebene senkrecht zur optischen Achse. In diesem Sinne können die Verriegelungsdrehachsen jeweils entweder einen Winkel ungleich 90° mit der optischen Achse einschließen oder windschief zur optischen Achse verlaufen, ohne dabei in einer Ebene senkrecht zur optischen Achse zu liegen. In der EP 2 401 953 B1 verlaufen die Verriegelungsdrehachsen der Verriegelungshebel nämlich in tangentialer Richtung und liegen in einer Ebene senkrecht zur optischen Achse.

Durch die erfindungsgemäße Schrägstellung der jeweiligen Verriegelungsdrehachse bezüglich der optischen Achse wird ein selbsthemmender Effekt erzielt, bei dem eine distalwärts gerichtete Zugkraft des Okulartrichters aus der Kupplungsvorrichtung gegen die Verriegelungselemente in der Verriegelungsposition dazu führt, dass die Verriegelungselemente stärker in die Verriegelungsposition gezogen werden. Die Kupplungsvorrichtung ist durch den selbsthemmenden Effekt auch toleranter gegenüber Verkantungen, Verkippungen oder Fertigungstoleranzen des Okulartrichters, da die Kontaktfläche oder der Kontaktpunkt zwischen dem Okulartrichter und den jeweiligen Verriegelungsabschnitten der Verriegelungselemente weniger genau bestimmt sein muss. Vorzugsweise entspricht die Form des Okulartrichters einer Norm, beispielsweise ISO/TS 18339, ISO 8600-2 oder ISO 8600-3, sodass verschiedenste endoskopische Systeme mittels der erfindungsgemäßen Kupplungsvorrichtung verbunden werden können. Es sei an dieser Stelle angemerkt, dass die Kupplungsvorrichtung an der proximalen Seite der Kupplungsvorrichtung mit einer Kamera nicht direkt verbunden oder lösbar verbindbar sein muss, sondern indirekt über einen Adapter und/oder ein Objektiv mit einer Kamera verbunden oder lösbar verbindbar sein kann. Vorzugsweise ist die Kupplungsvorrichtung allerdings fest mit einer Kamera verbunden bzw. fester Bestandteil einer Kamera.

Optional können die mindestens zwei Verriegelungselemente federnd zur Verriegelungsposition hin vorgespannt sein und sich vor Eindrücken eines Okulartrichters in die Okulartrichteraufnahme in der Verriegelungsposition befinden. Bei der EP 2 401 953 B1 ist dies genau andersherum, denn dort sind die Verriegelungsarme zur Freigabeposition hin federnd vorgespannt und befinden sich vor Eindrücken eines Okulartrichters in der Freigabeposition.

Optional können die mindestens zwei Verriegelungselemente dazu eingerichtet sein, durch Eindrücken eines Okulartrichters in die Okulartrichteraufnahme vom Okulartrichter zunächst kurzzeitig in die Freigabeposition gedrückt zu werden und dann selbsttätig den Okulartrichter in der Okulartrichteraufnahme verriegelnd in die Verriegelungsposition zurückzuschnappen. Dies ist besonders vorteilhaft, um einer Anwendungsperson ein haptisches und ein akustisches Feedback über eine erfolgreiche und korrekte Ankopplung des Okulartrichters zu geben.

Optional können die mindestens zwei Verriegelungselemente dazu eingerichtet sein, dass sich der Verriegelungsabschnitt jedes der Verriegelungselemente unter Drehung um die jeweils zugehörige Verriegelungsdrehachse von der Verriegelungsposition zur Freigabeposition schräg zur optischen Achse sowohl radial nach außen als auch proximalwärts bewegt. Durch die Schrägstellung der jeweiligen Verriegelungsdrehachse bewegt sich der jeweilige Verriegelungsabschnitt unter Drehung des Verriegelungselements sowohl radial nach außen als auch proximalwärts. Umgekehrt bewegt sich der jeweilige Verriegelungsabschnitt bei Drehung des Verriegelungselements von der Freigabeposition zur Verriegelungsposition sowohl radial nach innen als auch distalwärts. Zieht also der Okulartrichter aus der Kupplungsvorrichtung distalwärts heraus, während sich das Verriegelungselement in der Verriegelungsposition befindet, so zieht der Verriegelungsabschnitt das jeweilige Verriegelungselement stärker verriegelnd nach radial innen, so dass eine Selbsthemmung eintritt.

Optional kann die Kupplungsvorrichtung n ≥ 3 Verriegelungselemente aufweisen, wobei die Verriegelungselemente n-zählig rotationssymmetrisch bezüglich der optischen Achse angeordnet sind. Vorzugsweise weist die Kupplungsvorrichtung n = 3 Verriegelungselemente auf, die dreizählig rotationssymmetrisch bezüglich der optischen Achse angeordnet sind.

Optional kann die Kupplungsvorrichtung ferner ein manuell betätigbares Entriegelungselement aufweisen, wobei das Entriegelungselement mit jedem der Verriegelungselemente derart gekoppelt ist, dass jedes der Verriegelungselemente bei Betätigung des Entriegelungselements, vorzugsweise synchron mit den anderen Verriegelungselementen, radial nach außen aus der Verriegelungsposition in die Freigabeposition verschwenkt wird.

Optional kann das Entriegelungselement um die optische Achse drehbar in der Kupplungsvorrichtung gelagert sein und radial außenseitig der Kupplungsvorrichtung zum Drehen manuell greifbar sein. Im Gegensatz zu EP 2 401 953 B1 wird das Entriegelungselement vorzugsweise nicht axial proximalwärts gezogen, sondern hier um die optische Achse gedreht. Dies erleichtert eine Handhabung mit einer Hand und ist intuitiver.

Optional kann das Entriegelungselement eine um die optische Achse drehbar gelagerte Entriegelungsscheibe aufweisen, die für jedes der Verriegelungselemente zumindest einen ersten Koppelbereich zum Schwenken des jeweiligen Verriegelungselements zur Freigabeposition hin und vorzugsweise einen zweiten Koppelbereich zum Schwenken des jeweiligen Verriegelungselements zur Verriegelungsposition hin aufweist. Das Entriegelungselement kann also vorzugsweise auch dem Verriegeln dienen.

Optional kann das Entriegelungselement von einer Nominalstellung aus in eine erste Richtung in eine Freigabestellung und vorzugsweise von der Nominalstellung aus in eine der ersten Richtung entgegengesetzte zweite Richtung in eine Verklemmstellung bewegbar sein, wobei das Entriegelungselement in der Nominalstellung keine Kraft auf die Verriegelungselemente ausübt, in der Freigabestellung die Verriegelungselemente gegen eine Federkraft in die Freigabeposition radial nach außen drückt und vorzugsweise in der Verklemmstellung die Verriegelungselemente zur Erhöhung eines Kraftschlusses mit einem in der Okulartrichteröffnung verriegelnden Okulartrichters radial nach innen drückt.

Optional kann der Verriegelungsabschnitt jedes der Verriegelungselemente eine distalwärts weisende und zur optischen Achse hin abgeschrägt verlaufende Gleitfläche aufweisen. Die Gleitflächen dienen dazu, dass ein Okulartrichter beim Eindrücken des Okulartrichters in die Okulartrichteraufnahme der Kupplungsvorrichtung an den Gleitflächen entlang gleitet und dadurch die Verriegelungselemente radial nach außen zur Freigabeposition hindrückt. Die Verriegelungselemente bewegen sich dadurch automatisch und ohne weiteres zutun beim Eindrücken eines Okulartrichters in die Okulartrichteraufnahme der Kupplungsvorrichtung aus der Verriegelungsposition in die Freigabeposition.

Optional kann der Verriegelungsabschnitt jedes der Verriegelungselemente eine proximalwärts weisende Klemmfläche aufweisen, die in der Verriegelungsposition für einen selbsthemmenden Kraftschluss mit einem verriegelten Okulartrichter schräg zur optischen Achse hin gekippt ist. Der selbsthemmende Kraftschluss kann beispielsweise ein Reibschluss mit einer außenseitigen Mantelfläche des Okulartrichters sein. Die Verkippung der Klemmfläche schräg zur optischen Achse hin in der Verriegelungsposition kann vollständig durch die Schrägstellung der zugehörigen Verriegelungsdrehachse bedingt sein. In dem Fall kann die Klemmfläche senkrecht zur zugehörigen Verriegelungsdrehachse verlaufen. Alternativ oder zusätzlich dazu kann die Klemmfläche schräg zur zugehörigen Verriegelungsdrehachse verlaufen.

Optional kann die Gleitfläche des Verriegelungsabschnitts jedes der Verriegelungselemente zur optischen Achse hin steiler abgeschrägt sein als die Klemmfläche des Verriegelungsabschnitts jedes der Verriegelungselemente in der Verriegelungsposition zur optischen Achse hin gekippt ist. Dies ist vorteilhaft, um den Reibschluss an den Gleitflächen geringer auszugestalten als an den Klemmflächen.

Optional können die mindestens zwei Verriegelungselemente jeweils einen sich von der Verriegelungsdrehachse zum Verriegelungsabschnitt erstreckenden Hebelarm aufweisen, wobei sich der jeweilige Hebelarm in der Freigabeposition über das 0,1- bis 0,4-Fache eines inneren Umfangs der Okulartrichteraufnahme erstreckt. Im bevorzugten Fall von drei Verriegelungselementen, die dreizählig rotationsymmetrisch zur optischen Achse angeordnet sind, können die drei Verriegelungselemente jeweils einen sich von der Verriegelungsdrehachse zum Verriegelungsabschnitt erstreckenden Hebelarm aufweisen, wobei sich der Hebelarm in der Freigabeposition über das 0,1- bis 0,3-Fache des inneren Umfangs der Okulartrichteraufnahme erstreckt. Dadurch können die Hebelarme von drei Verriegelungselementen möglichst lang für einen selbsthemmenden Verriegelungseffekt ausgestaltet werden.

Optional kann die zu jedem der Verriegelungselemente zugehörige Verriegelungsdrehachse jeweils in Tangentialrichtung zur optischen Achse gekippt verlaufen. "In Tangentialrichtung zur optischen Achse gekippt" soll hier gerade nicht bedeuten, dass die Verriegelungsdrehachse selbst in Tangentialrichtung zur optischen Achse verläuft, sondern eine Parallele zur optischen Achse in einem Winkel ungleich 90° schneidet und mit dieser Parallelen eine Ebene aufspannt, die sich im Schnittpunkt in Tangentialrichtung zur optischen Achse erstreckt. Dies ist einerseits platzsparend in Radialrichtung und andererseits vorteilhaft, um ein distalwärtiges Anheben der Verriegelungsabschnitte von der Freigabeposition zur Verriegelungsposition zu erzielen.

Optional kann ein Gemeinlot zwischen der optischen Achse und der zu jedem der Verriegelungselemente gehörigen Verriegelungsdrehachse zwischen der proximalen Seite der Kupplungsvorrichtung und der distalen Seite der Kupplungsvorrichtung liegen. Vorzugsweise verläuft das Gemeinlot durch die Okulartrichteraufnahme. Bei leichter Verkippung der Verriegelungsdrehachse in Radialrichtung kann das Gemeinlot vorzugsweise noch zwischen der proximalen Seite der Kupplungsvorrichtung und der distalen Seite der Kupplungsvorrichtung liegen.

Die Erfindung wird im Folgenden detaillierter anhand der beiliegenden Zeichnungen erläutert. Es zeigen:
- Fig. 1: eine beispielhafte Ausführungsform eines endoskopischen Systems und eine Kamera mit einem Ausführungsbeispiel einer erfindungsgemäßen Kupplungsvorrichtung;
- Fig. 2: eine distalseitige perspektivische Ansicht eines Ausführungsbeispiels einer erfindungsgemäßen Kupplungsvorrichtung mit verriegeltem Okulartrichter;
- Fig. 3a,b: distalseitige perspektivische Ansichten der Ausführungsform einer erfindungsgemäßen Kupplungsvorrichtung gemäß Fig. 2 einmal ohne verriegelten Okulartrichter und einmal mit verriegeltem Okulartrichter und weggelassenem Ringgehäuse;
- Fig. 4a-c: verschiedene halbtransparente Ansichten des Ausführungsbeispiels der Kupplungsvorrichtung gemäß Fig. 1, 2 und 3a,b mit Verriegelungselementen in der Verriegelungsposition;
- Fig. 5a,b: zwei halbtransparente Ansichten der Ausführungsform gemäß Fig. 4a-c mit Verriegelungselementen in der Freigabeposition;
- Fig. 6a,b: proximalseitige perspektivische Ansichten einer Ausführungsform der erfindungsgemäßen Kupplungsvorrichtung mit einer ersten Ausführungsform eines Entriegelungselements einmal mit Verriegelungselementen in der Freigabeposition und einmal mit Verriegelungselementen in der Verriegelungsposition;
- Fig. 7, 8a,b: verschiedene Ansichten einer Ausführungsform der erfindungsgemäßen Kupplungsvorrichtung mit einer zweiten Ausführungsform eines Entriegelungselements; und
- Fig. 9: eine proximalseitige Ansicht einer Ausführungsform der erfindungsgemäßen Kupplungsvorrichtung mit einer dritten Ausführungsform eines Entriegelungselements.

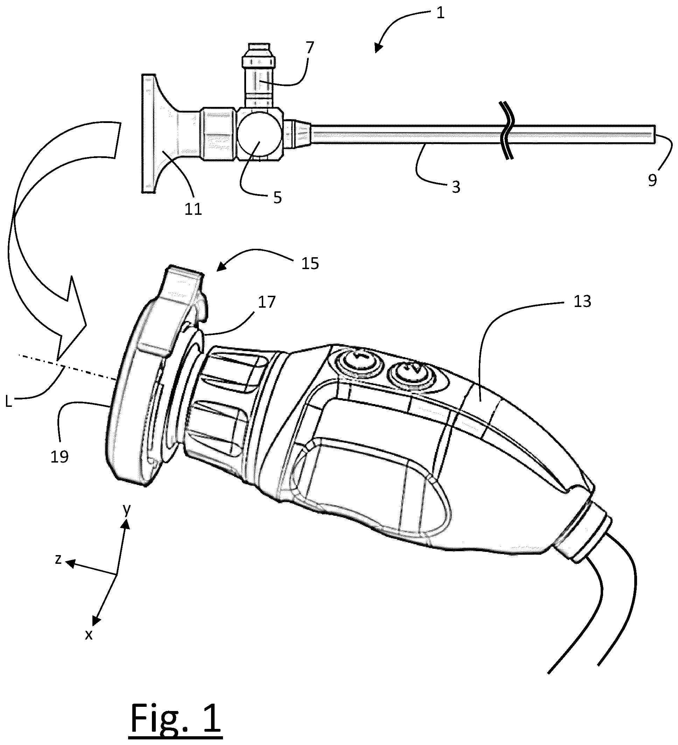

Fig. 1 zeigt ein Ausführungsbeispiel eines endoskopischen Systems 1, das zur endoskopischen medizinischen Untersuchung eines Patienten geeignet ist. Das endoskopische System 1 weist einen länglichen Einführabschnitt 3 auf, der zumindest teilweise in das Körperinnere eines Patienten einführbar ist. An einem proximalen Ende des Einführabschnitts 3 ist ein Strahlteiler 5 angeordnet, mit dem seitlich über einen Lichtquellenanschluss 7 Licht in den Einführabschnitt 3 eingekoppelt werden kann. Ein sich durch den Einführabschnitt 3 erstreckender Lichtleiter leitet das eingekoppelte Licht bis an ein distales Ende 9 des Einführabschnitts 3, wo das Licht zur Beleuchtung ausgekoppelt wird. Proximal vom Strahlteiler 5 ist ein Okulartrichter 11 des endoskopischen Systems 1 angeordnet, der das proximale Ende des endoskopischen Systems 1 bildet. Es sei an dieser Stelle angemerkt, dass sich die Begriffe "proximal" und "distal" nicht auf den zu untersuchenden Patienten beziehen, sondern auf die Anwendungsperson. Eine Anwendungsperson kann ihr Auge an den Okulartrichter 11 ansetzen und so durch das endoskopische System 1 in das Körperinnere des Patienten sehen.

Zur Aufnahme von Bildern oder Videos mittels des endoskopischen Systems 1 kann der Okulartrichter 11 an eine Kamera 13 angeschlossen werden. Dazu ist an einem distalen Ende der Kamera 13 eine Kupplungsvorrichtung 15 angeschlossen, die zum lösbaren Anschließen des Okulartrichters 11 des endoskopischen Systems 1 an die Kamera 13 dient. Die Kupplungsvorrichtung 15 kann mit einer proximalen Seite 17 der Kupplungsvorrichtung 15 mit der Kamera 13 fest verbunden oder lösbar verbindbar sein. Die Kamera 13 definiert dabei eine optische Achse L, die sich distalwärts von der Kamera 13 erstreckt. Die Kupplungsvorrichtung 15 ist koaxial zur optischen Achse L angeordnet, so dass das mittels der Kupplungsvorrichtung 15 an die Kamera 13 angeschlossene endoskopische System 1 koaxial zur optischen Achse L verläuft. Zur besseren Orientierung in den Figuren weisen die Figuren jeweils ein rechtshändiges kartesisches Koordinatensystem auf, wobei die z-Achse in Richtung der optischen Achse L distalwärts verläuft. Die x-Achse und die y-Achse spannen eine zu der in z-Richtung verlaufende optische Achse L senkrechte Radialebene auf. Die Begriffe "axial" und "radial" sind hier also bezüglich der optischen Achse L zu verstehen.

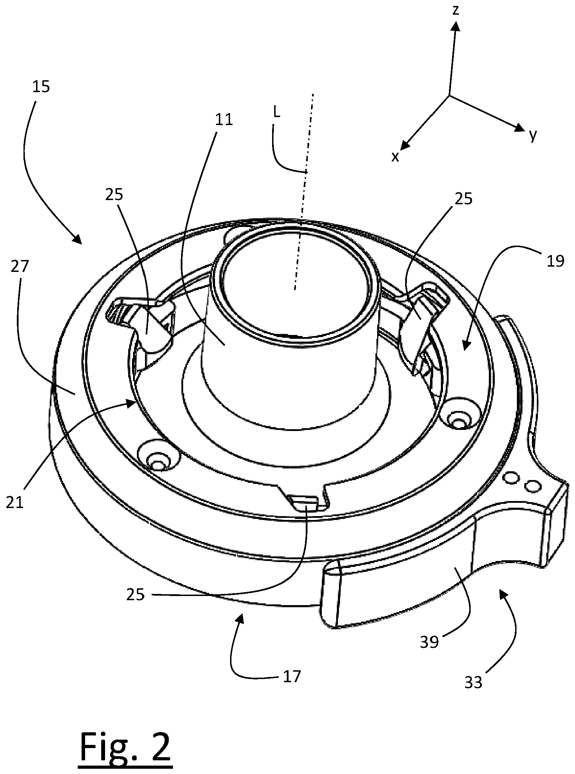

Fig. 2 zeigt eine perspektivische Ansicht auf eine distale Seite 19 der Kupplungsvorrichtung 15 mit einem darin verriegelten Okulartrichter 11. An der distalen Seite 19 weist die Kupplungsvorrichtung 15 eine Okulartrichteraufnahme 21 in Form einer axialen Vertiefung auf, die koaxial zur optischen Achse L angeordnet ist und in welcher der Okulartrichter 11 des endoskopischen Systems 1 verriegelt ist. Der Innendurchmesser der Okulartrichteraufnahme 21 ist an den Außendurchmesser des Okulartrichters 11 angepasst und etwas größer, damit der Okulartrichter 11 in die Okulartrichteraufnahme 21 passt. Die Kupplungsvorrichtung 15 in Fig. 2 weist drei nur teilweise sichtbare Verriegelungselemente 23 auf, die jeweils einen Verriegelungsabschnitt 25 zum Verriegeln des Okulartrichters 11 in der Okulartrichteraufnahme 21 aufweisen. Die Verriegelungselemente 23 sind zwischen einer Freigabeposition und einer Verriegelungsposition um eine dem jeweiligen Verriegelungselement 25 zugehörige Verriegelungsdrehachse A (in Fig. 2 nicht sichtbar) drehbar gelagert. In Fig. 2 befinden sich die Verriegelungselemente 23 in der Verriegelungsposition, in der der jeweilige Verriegelungsabschnitt 25 in die Okulartrichteraufnahme 21 ragt und dadurch den Okulartrichter 11 in der Okulartrichteraufnahme 21 verriegelt.

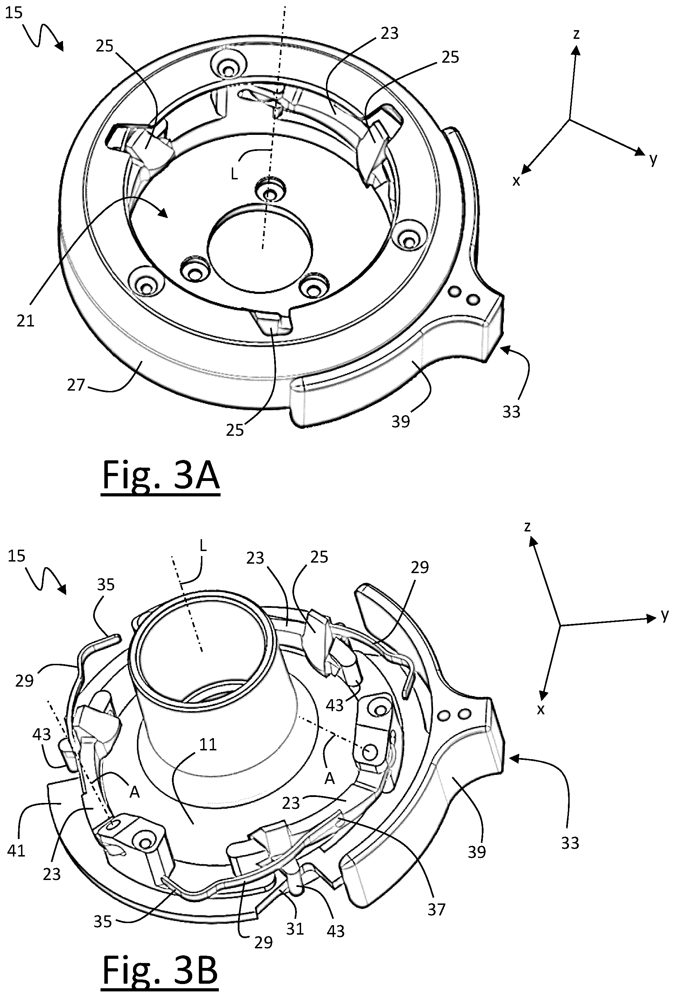

Fig. 3a zeigt den Verriegelungsmechanismus deutlicher ohne den Okulartrichter 11. In Fig. 3b ist zur Verdeutlichung des Verriegelungsmechanismus ein Ringgehäuse 27 der Kupplungsvorrichtung 15 nicht gezeigt. In Fig. 3a,b sind die Verriegelungselemente 23 in Form von Hebelarmen sichtbar, deren Länge sich ungefähr über das 0,2-fache eines inneren Umfangs der Okulartrichteraufnahme 21 erstreckt. Der jeweilige Verriegelungsabschnitt 25 wird von einem freien Ende des Verriegelungselements 23 gebildet. Das andere Ende des Verriegelungselements 23 bildet die zugehörige Verriegelungsdrehachse A oder ist an dieser angelenkt. Die zu jedem der Verriegelungselemente 23 zugehörige Verriegelungsdrehachse A verläuft jeweils derart windschief zur optischen Achse L, dass sich ein jeweiliges Gemeinlot zwischen optischer Achse L und Verriegelungsdrehachse A durch die Okulartrichteraufnahme 21 erstreckt. Die Verriegelungsdrehachse A ist entsprechend gegenüber der optischen Achse in Tangentialrichtung verkippt. Die Anordnung der drei Verriegelungselemente 23 bezüglich der optischen Achse L ist dreizählig rotationssymmetrisch.

In Fig. 3b wird deutlich, dass die Verriegelungselemente 23 jeweils federnd zur Verriegelungsposition hin vorgespannt sind und sich vor Eindrücken des Okulartrichters 11 in die Okulartrichteraufnahme 21 in der Verriegelungsposition, wie in Fig. 2, 3a und 3b gezeigt, befinden. Zur federnden Vorspannung der Verriegelungselemente 23 weist die Kupplungsvorrichtung 15 entsprechende Federelemente 29 auf. Die Federelemente 29 drücken die Verriegelungselemente 23 radial nach innen, so dass sie um die jeweilige Verriegelungsdrehachse A so weit wie möglich nach innen gedrückt werden. Die Bewegung der Verriegelungselemente 23 nach radial innen ist entweder dadurch beschränkt, dass die jeweiligen Verriegelungsabschnitte 25 in einen Reibschluss mit dem Okulartrichter 11 geraten oder, wenn kein Okulartrichter 1 eingedrückt ist, gegen einen ersten Koppelbereich 31 eines Entriegelungselements 33 stoßen.

Aus den Fig. 3a,b wird deutlich, dass die drei Verriegelungselemente 23 jeweils dazu eingerichtet sind, durch Eindrücken des Okulartrichters 11 in die Okulartrichteraufnahme 21 vom Okulartrichter 11 zunächst kurzzeitig in die Freigabeposition gedrückt zu werden und dann selbsttätig den Okulartrichter 11 in der Okulartrichteraufnahme 21 verriegelnd in die Verriegelungsposition zurückzuschnappen. Durch die Schrägstellung der Verriegelungsdrehachse A bewegt sich der Verriegelungsabschnitt 25 jedes der Verriegelungselemente 23 unter Drehung um die jeweils zugehörige Verriegelungsdrehachse A von der Verriegelungsposition zur Freigabeposition schräg zur optischen Achse L sowohl radial nach außen als auch proximalwärts, also zur proximalen Seite 17 der Kupplungsvorrichtung 15 hin.

In Fig. 3b wird deutlich, dass die Kupplungsvorrichtung 15 in dem gezeigten Ausführungsbeispiel drei Verriegelungselemente 23 aufweist, die dreizählig rotationssymmetrisch bezüglich der optischen Achse L angeordnet sind. Die Federelemente 29 erstrecken sich radial außenseitig der jeweiligen Verriegelungselemente 23 in tangentialer Verlängerung der Verriegelungselemente 23 über die Länge der Verriegelungselemente 23 hinaus und stützen sich mit einem freien Ende 35 federnd gegen eine radiale Innenwandung des Ringgehäuses 27 ab. Dadurch spannt das Federelement 29 mit seinem mit dem Verriegelungselement 23 verbundenen Ende 37 das Verriegelungselement 23 nach radial innen vor. Es sind allerdings auch andere Formen und Anordnungen von Federelementen denkbar, um die Verriegelungselemente 23 nach radial innen vorzuspannen.

Fig. 3b zeigt außerdem deutlich die Funktionsweise des Entriegelungselements 33 der Kupplungsvorrichtung 15. Das Entriegelungselement 33 weist radial außenseitig vom Ringgehäuse 27 ein Griffteil 39 auf, das mit einer Entriegelungsscheibe 41 des Entriegelungselements 33 verbunden oder damit einstückig ausgebildet ist. Die Entriegelungsscheibe 41 ist um die optische Achse L drehbar gelagert und kann mittels des Griffteils 39 um die optische Achse L gedreht werden. Die Verriegelungselemente 23 weisen jeweils einen proximalwärts verlaufenden Führungsstift 43 auf, der am ersten Koppelbereich 31 der Entriegelungsscheibe 41 gleitend anliegt. Durch rechtshändige Drehung der Entriegelungsscheibe 41 um die in z-Richtung verlaufende optische Achse L gleitet der Führungsstift 43 des Verriegelungselements 23 den ersten Koppelbereich 31 entlang und schwenkt dabei das Verriegelungselement 23 radial nach außen zur Freigabeposition hin. Der Verlauf des ersten Koppelbereichs 31 der Entriegelungsscheibe 41 kann so gewählt werden, dass er einerseits flach genug verläuft, um mit entsprechender Kraftübertragung das Verriegelungselement 23 gegen die Federkraft des Federelements 29 zur Freigabeposition hin nach radial außen zu drücken. Andererseits kann der erste Koppelbereich 31 vorzugsweise so steil ausgestaltet sein, dass die Federkraft des Federelements 29 ausreicht, um die Entriegelungsscheibe 41 selbsttätig in eine Nominalstellung zurückzudrücken, wenn eine Anwendungsperson das Griffteil 39 loslässt. Das automatisch in die Nominalstellung zurückschnappende Griffteil 39 gibt einer Anwendungsperson ein optisches Feedback darüber, dass die Verriegelungselemente 23 erfolgreich automatisch den Okulartrichter 11 verriegelt haben.

In Fig. 3b ist nicht gezeigt, dass die Entriegelungsscheibe 41 vorzugsweise auch einen zweiten Koppelbereich 49 aufweisen kann, um das jeweilige Verriegelungselement 23 zur Verriegelungsposition hin zu verschwenken. Ein solcher zweiter Koppelbereich 49 könnte beispielsweise durch einen Schlitz 51 in der Entriegelungsscheibe 41 erzielt werden, der den Führungsstift 43 auch radial außenseitig führt. Beispielsweise durch aktives Drücken des Griffteils 39 entgegengesetzt zur Entriegelungsrichtung könnte ein solcher zweiter Koppelbereich 49 das jeweilige Verriegelungselement 23 aktiv mit manueller Kraft nach radial innen drücken, um beispielsweise den Reibschluss zwischen den Verriegelungsabschnitten 25 und dem Okulartrichter 11 zu erhöhen. Das Entriegelungselement 23 mit dem Griffteil 39 und der Entriegelungsscheibe 41 könnte dann in einer solchen Verklemmstellung feststecken und müsste manuell durch Betätigung des Griffteils 39 wieder in die Nominalstellung oder in die Freigabestellung gebracht werden.

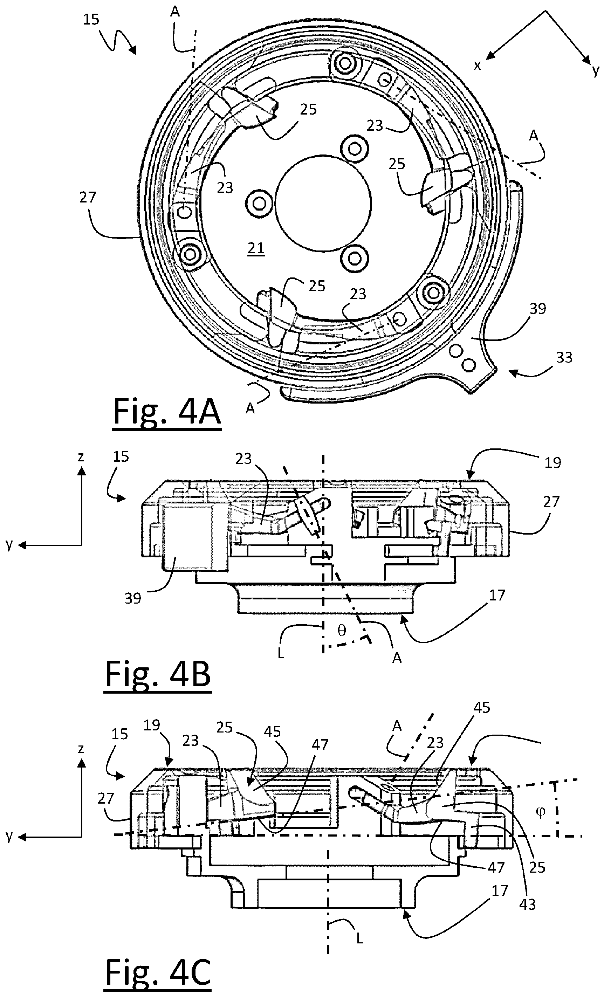

In Fig. 4a-c, die in verschiedenen halbtransparenten Ansichten die Kupplungsvorrichtung 15 mit den Verriegelungselementen 23 in der Verriegelungsposition zeigen, wird insbesondere die Schrägstellung der jeweiligen Verriegelungsdrehachsen A verdeutlicht. Die Verriegelungsdrehachse A ist in dem gezeigten Ausführungsbeispiel um 15° bis 40° windschief zur optischen Achse L schräggestellt, wobei sich das Gemeinlot zwischen optischer Achse L und Verriegelungsdrehachse A durch die Okulartrichteraufnahme 21 erstreckt. Die Verriegelungsdrehachse A ist entsprechend gegenüber der optischen Achse in Tangentialrichtung verkippt. Im gezeigten Ausführungsbeispiel beträgt die Schrägstellung in etwa 25°, welche in Fig. 4b durch den Winkel θ dargestellt ist. In Fig. 4c ist erkennbar, dass der Verriegelungsabschnitt 25 in der Verriegelungsposition des Verriegelungselements 23 eine maximal distale Position hat und gerade so ausgestaltet ist, dass der Verriegelungsabschnitt 25 nicht über die distale Seite 19 der Kupplungsvorrichtung 15 hinausragt.

Der Verriegelungsabschnitt 25 jedes der Verriegelungselemente 23 weist eine distalwärts zur optischen Achse L abgeschrägt verlaufende Gleitfläche 45 auf. Die Gleitfläche 45 ist so abgeschrägt, dass ein distales Ende der Gleitfläche 45 selbst in der gezeigten Verriegelungsposition der Verriegelungselemente 23 radial außerhalb der Okulartrichteraufnahme 21 verbleibt. Die Gleitfläche 45 verläuft vom distalen Ende aus schräg sowohl proximalwärts als auch nach radial innen, so dass der Okulartrichter 11 beim manuellen Eindrücken des Okulartrichters 11 in die Okulartrichteraufnahme 21 an den Gleitflächen 45 entlanggleitet und die Verriegelungselemente 23 dadurch nach radial außen in die Freigabeposition drückt.

Unterseitig, also zur proximalen Seite 17 der Kupplungsvorrichtung 15 hin, weist der Verriegelungsabschnitt 25 jedes der Verriegelungselemente 23 eine proximalwärts weisende Klemmfläche 47 auf, die in der Verriegelungsposition zur Erzielung eines selbsthemmenden Kraftschlusses mit dem verriegelten Okulartrichter 11 schräg zur optischen Achse L hin gekippt ist. Dies ist in Fig. 4c gut zu erkennen, wenngleich der Winkel φ der Verkippung der Klemmfläche 47 gegenüber einer zur optischen Achse L senkrechten Fläche relativ klein ist, vorzugsweise unterhalb von 10°. In Fig. 4c ist die Verkippung der Klemmfläche 47 in der gezeigten Verriegelungsposition durch den Winkel φ gezeigt. Der Verkippwinkel φ kann sich mit der Position des Verriegelungselements 23 ändern und in der Freigabeposition sogar 0° betragen. Vorzugsweise ist die Gleitfläche 45 des Verriegelungsabschnitts 25 jedes der Verriegelungselemente 23 zur optischen Achse L hin steiler abgeschrägt als die Klemmfläche 47 des Verriegelungsabschnitts 25 jedes der Verriegelungselemente 23 in der Verriegelungsposition der optischen Achse L hin gekippt ist.

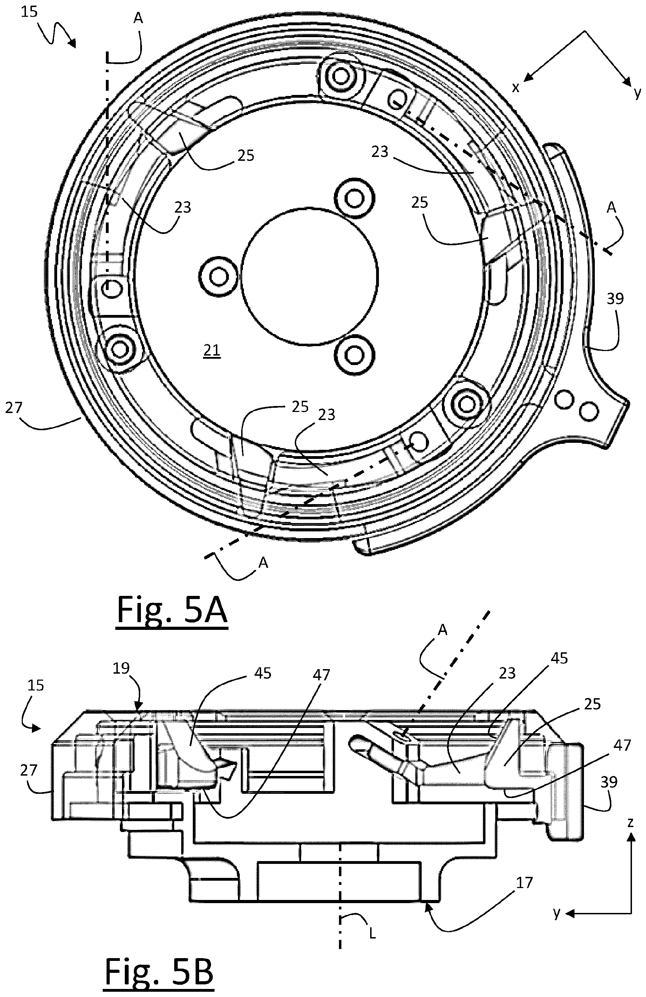

In Fig. 5a,b sind die Verriegelungselemente 23 in der Freigabeposition gezeigt. In Fig. 5b ist gut erkennbar, dass sich der Verriegelungsabschnitt 25 sowohl nach radial außen als auch proximalwärts bewegt hat und die Klemmfläche 47 in der gezeigten Freigabeposition in etwa senkrecht zur optischen Achse L verläuft.

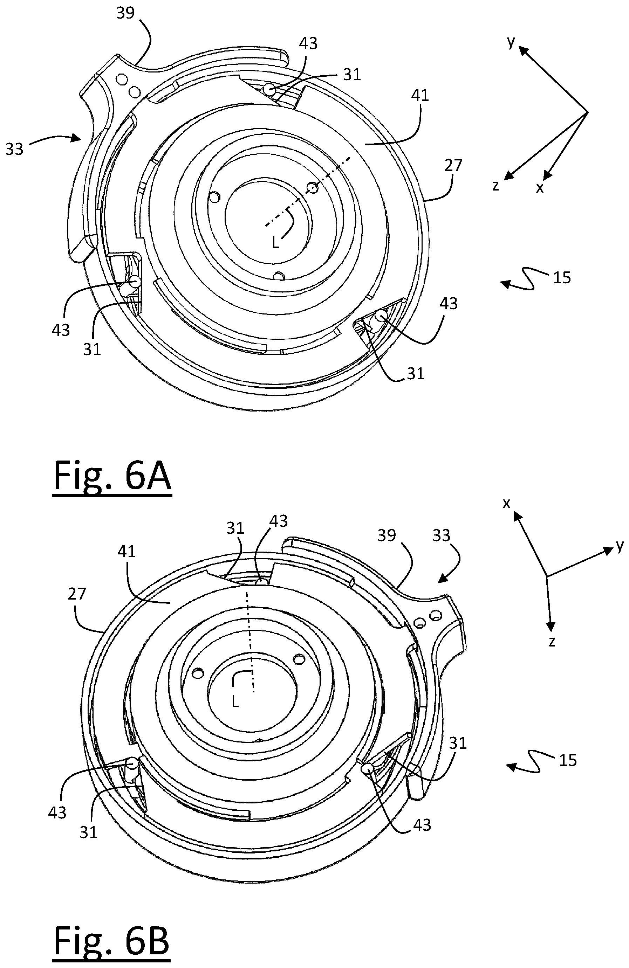

In Fig. 6a,b, die die Kupplungsvorrichtung 15 von der proximalen Seite 17 zeigen, an welche die Kamera 13 anschließbar ist, verdeutlichen noch einmal die Funktionsweise des Entriegelungselements 33. Die Entriegelungsscheibe 41 des Entriegelungselements 33 weist eine radiale Auskragung auf, die radial nach außen durch eine radiale Aussparung im Ringgehäuse 27 ragt, und dadurch mit dem Griffteil 39 des Entriegelungselements 33 verbunden ist, so dass sich die Entriegelungsscheibe 41 durch manuelles Betätigen des Griffteils 39 um die optische Achse L drehen lässt. Die schräg verlaufenden ersten Koppelbereiche 31 der Entriegelungsscheibe 41 drücken die Führungsstifte 43 der Verriegelungselemente 23 radial nach außen, wenn das Griffteil 39 manuell aus einer in Fig. 6b gezeigten Nominalstellung in eine in Fig. 6a gezeigte Freigabestellung gedrückt wird. Dementsprechend befinden sich die Verriegelungselemente 23 in der Fig. 6a in der Freigabeposition und in der Fig. 6b in der Verriegelungsposition.

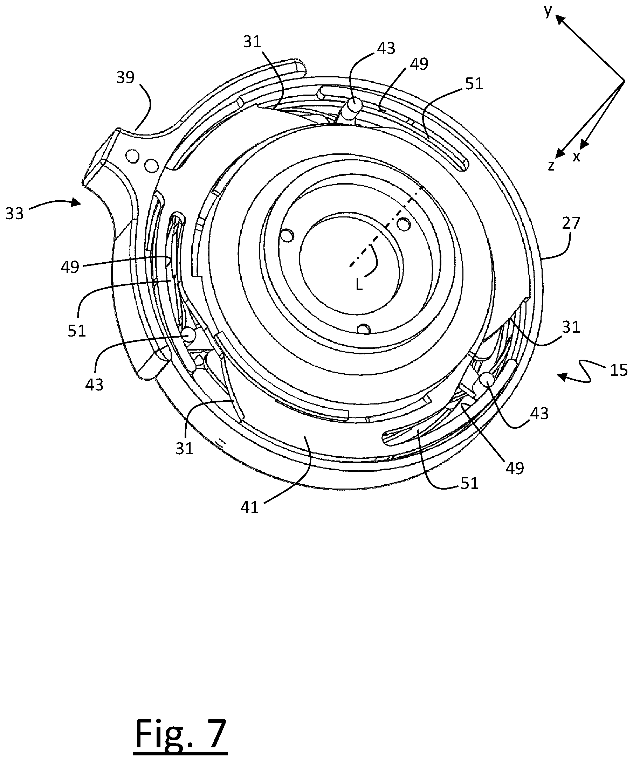

Fig. 7 zeigt eine Ausführungsform der Entriegelungsscheibe 41 mit einem zweiten Koppelbereich 49, mit dem die Entriegelungsscheibe 41 die Führungsstifte 43 der Verriegelungselemente 23 nach radial innen zur Verriegelungsposition hin drücken kann. Das Entriegelungselement 33 ist hier von einer Nominalstellung aus in eine erste Richtung, hier rechtshändig im Uhrzeigersinn um eine in z-Richtung verlaufende Drehachse in eine Freigabestellung und von der Nominalstellung aus in eine der ersten Richtung entgegengesetzte zweite Richtung, hier rechtshändig gegen den Uhrzeigersinn um eine in z-Richtung verlaufende Drehachse, in eine Verklemmstellung bewegbar, wobei das Entriegelungselement 33 in der Nominalstellung keine Kraft auf die drei Verriegelungselemente 23 ausübt, in der Freigabestellung die drei Verriegelungselemente 23 gegen eine Federkraft in die Freigabeposition radial nach außen drückt und in der Verklemmstellung die drei Verriegelungselemente 23 zur Erhöhung eines Kraftschlusses mit dem in der Okulartrichteröffnung 21 verriegelten Okulartrichter 11 radial nach innen drückt. Es sei an dieser Stelle angemerkt, dass Fig. 7 die Verriegelungselemente 23 gerade in einer momentanen Freigabestellung zeigt, die nicht durch das Entriegelungselement 33 verursacht wird, sondern durch den Okulartrichter 11, der gerade in die Okulartrichteröffnung 21 eingedrückt wird. Durch die Federkraft der Federelemente 29 schnappen die Verriegelungselemente 23 mit den Führungsstiften 43 jeden Moment radial nach innen, um an den ersten Koppelbereich 31 der Entriegelungsscheibe 41 anzustoßen und/oder damit die Klemmflächen 47 in einen selbsthemmenden Reibschluss mit dem Okulartrichter 11 kommen. Zur Verklemmstellung hin bilden der erste Koppelbereich 31 und der zweite Koppelbereich 49 der Entriegelungsscheibe 41 einen dazwischenliegenden Führungsschlitz 51, in welchen der jeweilige Führungsstift 43 zur Verklemmung der Verriegelungselemente 23 in der Verklemmstellung geführt ist. Das Entriegelungselement 33 verbleibt ohne manuellen Eingriff in der Verklemmstellung, wohingegen es automatisch aus der Freigabestellung zurück in die Nominalstellung zurückschnappt, wenn die Anwendungsperson das Griffteil 39 loslässt. Es kann auch vorgesehen werden, dass eine Anwendungsperson das Griffteil 39 in der Verklemmstellung fixieren kann, beispielsweise durch Reib- und/oder Formschluss des Führungsstifts 43 mit der Entriegelungsscheibe 41.

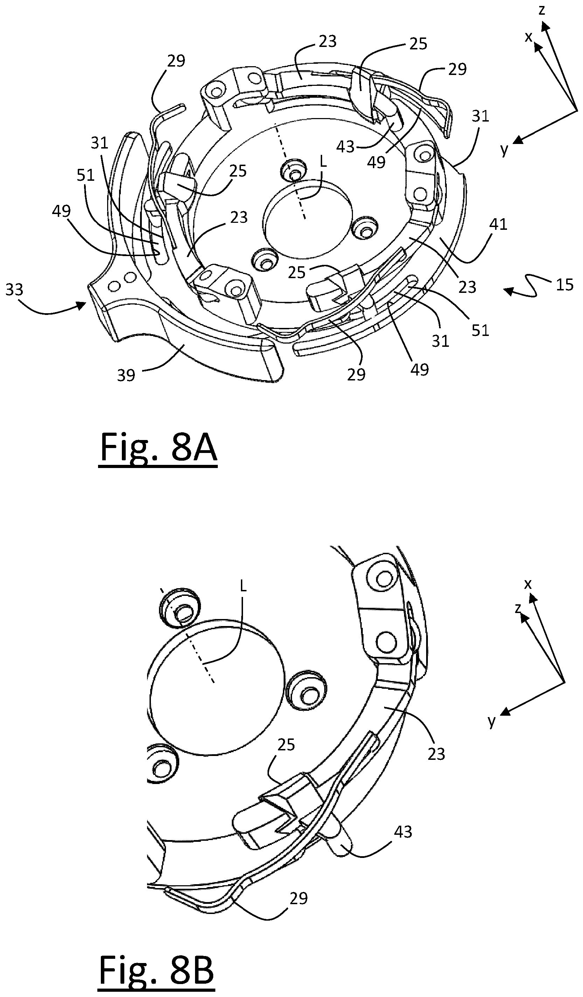

Fig. 8a,b zeigen das in Fig. 7 gezeigte Ausführungsbeispiel der Verriegelungselemente 23 in der verklemmten Verriegelungsposition.

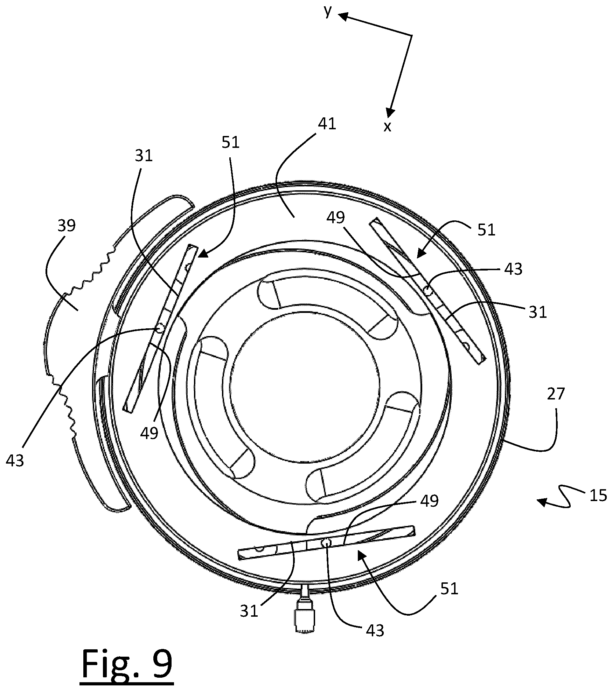

In Fig. 9 ist eine weitere Ausführungsform der Entriegelungsscheibe 41 gezeigt, bei der die Führungsstifte 43 jeweils in einem gradlinig von einer gezeigten Nominalstellung in tangentiale Richtung verlaufenden Führungsschlitz 51 geführt ist. Diese Ausführungsform hat den Vorteil, dass eine Anwendungsperson das Griffteil 39 in eine beliebige Richtung betätigen kann, um die Verriegelungselemente 23 in die Freigabeposition zu bewegen. Eine radiale Innenseite des Führungsschlitzes 51 bildet dabei den ersten Koppelbereich 31 und eine radiale Außenseite des Führungsschlitzes 51 bildet den zweiten Koppelbereich 49. Ein automatisches Öffnen der Verriegelungselemente 23 durch Eindrücken des Okulartrichters 11 in die Okulartrichteraufnahme 21 ist hier nicht möglich, sondern das Entriegelungselement 33 muss manuell betätigt werden, um die Kupplungsvorrichtung 15 zu öffnen. Durch Federkraft der Federelemente 29 schnappen dann die Verriegelungselemente 23 verriegelnd zu, wenn das Griffelement 39 losgelassen wird. Kommen die Verriegelungsstifte 43 beim verriegelnden Zurückschnappen nicht in die in Fig. 9 gezeigte Nominalstellung, weil die Klemmflächen 47 in einen selbsthemmenden Reibschluss mit dem Okulartrichter 11 kommen, so kann eine Anwendungsperson das Griffteil 39 betätigen, um das Entriegelungselement 33 in die gezeigte Nominalstellung zu bringen und somit durch den zweiten Koppelbereich 49 die Führungsstifte 43 und damit die jeweiligen Verriegelungselemente 23 nach radial innen zu drücken und somit den Reibschluss der Klemmfläche 47 mit dem verriegelten Okulartrichter 11 zu erhöhen.

### Bezugszeichenliste

- 1: Endoskopisches System
- 3: Einführabschnitt
- 5: Strahlteiler
- 7: Lichtquellenanschluss
- 9: distales Ende des Einführabschnitts
- 11: Okulartrichter
- 13: Kamera
- 15: Kupplungsvorrichtung
- 17: proximale Seite der Kupplungsvorrichtung
- 19: distale Seite der Kupplungsvorrichtung
- 21: Okulartrichteraufnahme
- 23: Verriegelungselemente
- 25: Verriegelungsabschnitte der Verriegelungselemente
- 27: Ringgehäuse
- 29: Federelemente
- 31: erster Koppelbereich
- 33: Entriegelungselement
- 35: freies Ende der Federelemente
- 37: ein mit dem Verriegelungselement verbundene Ende der Federelemente
- 39: Griffteil
- 41: Entriegelungsscheibe
- 43: Führungsstift
- 45: Gleitfläche
- 47: Klemmfläche
- 49: zweiter Koppelbereich
- 51: Führungsschlitz
- L: optische Achse
- A: Verriegelungsdrehachse
- θ: Schrägstellungswinkel
- φ: Kippwinkel

## Patentansprüche

1. Kupplungsvorrichtung (15) zum lösbaren Anschließen eines Okulartrichters (11) eines endoskopischen Systems (1) an eine Kamera (13), wobei die Kupplungsvorrichtung (15) an einer proximalen Seite (17) der Kupplungsvorrichtung (15) mit einer Kamera (13) verbunden oder lösbar verbindbar ist, wobei die Kupplungsvorrichtung (15) an einer in Richtung einer optische Achse (L) der proximalen Seite (17) der Kupplungsvorrichtung (15) gegenüberliegenden distalen Seite (19) der Kupplungsvorrichtung (15) mit einem Okulartrichter (11) lösbar verbindbar ist, wobei die Kupplungsvorrichtung (15) aufweist:
- eine an derdistalen Seite (19) der Kupplungsvorrichtung (15) angeordnete Okulartrichteraufnahme (21), in welche ein Okulartrichter (11) eines endoskopischen Systems (1) manuell gedrückt werden kann, und
- mindestens zwei Verriegelungselemente (23) mit jeweils einem Verriegelungsabschnitt (25) zum Verriegeln eines Okulartrichters (11) in der Okulartrichteraufnahme (21), wobei die mindestens zwei Verriegelungselemente (23) jeweils zwischen einer Freigabeposition und einer Verriegelungsposition um eine dem jeweiligen Verriegelungselement (23) zugehörige Verriegelungsdrehachse (A) drehbar gelagert sind,
wobei der jeweilige Verriegelungsabschnitt (25) der mindestens zwei Verriegelungselemente (23) in der Verriegelungsposition des jeweiligen Verriegelungselements (23) in die Okulartrichteraufnahme (21) ragt und sich in der Freigabeposition des jeweiligen Verriegelungselements (23) radial außerhalb der Okulartrichteraufnahme (21) befindet, wobei die zu jedem der Verriegelungselemente (23) zugehörige Verriegelungsdrehachse (A) jeweils schräg zur optischen Achse (L) verläuft.

2. Kupplungsvorrichtung (15) nach Anspruch 1, wobei die mindestens zwei Verriegelungselemente (23) federnd zur Verriegelungsposition hin vorgespannt sind und sich vor Eindrücken eines Okulartrichters (11) in die Okulartrichteraufnahme (21) in der Verriegelungsposition befinden.

3. Kupplungsvorrichtung (15) nach Anspruch 1 oder 2, wobei die mindestens zwei Verriegelungselemente (23) dazu eingerichtet sind, durch Eindrücken eines Okulartrichters (11) in die Okulartrichteraufnahme (21) vom Okulartrichter (11) zunächst kurzzeitig in die Freigabeposition gedrückt zu werden und dann selbsttätig den Okulartrichter (11) in der Okulartrichteraufnahme (21) verriegelnd in die Verriegelungsposition zurückzuschnappen.

4. Kupplungsvorrichtung (15) nach einem der vorhergehenden Ansprüche, wobei die mindestens zwei Verriegelungselemente (23) dazu eingerichtet sind, dass sich der Verriegelungsabschnitt jedes der Verriegelungselemente (25) unter Drehung um die jeweils zugehörige Verriegelungsdrehachse (A) von der Verriegelungsposition zur Freigabeposition schräg zur optischen Achse (L) sowohl radial nach außen als auch zur proximalen Seite (17) der Kupplungsvorrichtung (15) hin bewegt.

5. Kupplungsvorrichtung (15) nach einem der vorhergehenden Ansprüche, wobei die Kupplungsvorrichtung (15) mindestens *n* ≥ 3 Verriegelungselemente (23) aufweist, wobei die Verriegelungselemente (23) n-zählig rotationssymmetrisch bezüglich der optischen Achse (L) angeordnet sind.

6. Kupplungsvorrichtung (15) nach einem der vorhergehenden Ansprüche, wobei die Kupplungsvorrichtung (15) ferner ein manuell betätigbares Entriegelungselement (33) aufweist, wobei das Entriegelungselement (33) mit jedem der Verriegelungselemente (23) derart gekoppelt ist, dass jedes der Verriegelungselemente (23) bei Betätigung des Entriegelungselements (33), vorzugsweise synchron mit den anderen Verriegelungselementen (23), radial nach außen aus der Verriegelungsposition in die Freigabeposition verschwenkt wird.

7. Kupplungsvorrichtung (15) nach Anspruch 6, wobei das Entriegelungselement (33) um die optische Achse (L) drehbar in der Kupplungsvorrichtung (15) gelagert ist und radial außenseitig der Kupplungsvorrichtung (15) zum Drehen manuell greifbar ist.

8. Kupplungsvorrichtung (15) nach Anspruch 6 oder 7, wobei das Entriegelungselement (33) eine um die optische Achse (L) drehbar gelagerte Entriegelungsscheibe (41) aufweist, die für jedes der Verriegelungselemente (23) zumindest einen ersten Koppelbereich (31) zum Schwenken des jeweiligen Verriegelungselements (23) zur Freigabeposition hin und vorzugsweise einen zweiten Koppelbereich (49)zum Schwenken des jeweiligen Verriegelungselements (23) zur Verriegelungsposition hin aufweist.

9. Kupplungsvorrichtung (15) nach einem der Ansprüche 6 bis 8, wobei das Entriegelungselement (33) von einer Nominalstellung aus in eine erste Richtung in eine Freigabestellung und vorzugsweise von der Nominalstellung aus in eine der ersten Richtung entgegengesetzte zweite Richtung in eine Verklemmstellung bewegbar ist, wobei das Entriegelungselement (33) in der Nominalstellung keine Kraft auf die Verriegelungselemente (23) ausübt, in der Freigabestellung die Verriegelungselemente (23) gegen eine Federkraft in die Freigabeposition radial nach außen drückt und vorzugsweise in der Verklemmstellung die Verriegelungselemente (23) zur Erhöhung eines Kraftschlusses mit einem in der Okulartrichteröffnung verriegelten Okulartrichters (11) radial nach innen drückt.

10. Kupplungsvorrichtung (15) nach einem der vorhergehenden Ansprüche, wobei der Verriegelungsabschnitt (25) jedes der Verriegelungselemente (23) eine distalwärts weisende und zur optischen Achse (L) hin abgeschrägt verlaufende Gleitfläche (45) aufweist.

11. Kupplungsvorrichtung (15) nach einem der vorhergehenden Ansprüche, wobei der Verriegelungsabschnitt (25) jedes der Verriegelungselemente (23) eine proximalwärts weisende Klemmfläche (47) aufweist, die in der Verriegelungsposition für einen selbsthemmenden Kraftschluss mit einem verriegelten Okulartrichter (11) schräg zur optischen Achse (L) hin gekippt ist.

12. Kupplungsvorrichtung (15) nach Anspruch 10 und 11, wobei die Gleitfläche (45) des Verriegelungsabschnitts (25) jedes der Verriegelungselemente (23) zur optischen Achse (L) hin steiler abgeschrägt ist als die Klemmfläche (47) des Verriegelungsabschnitts (25) jedes der Verriegelungselemente (23) in der Verriegelungsposition zur optischen Achse (L) hin gekippt ist.

13. Kupplungsvorrichtung (15) nach einem der vorhergehenden Ansprüche, wobei die mindestens zwei Verriegelungselemente (23) jeweils einen sich von der Verriegelungsdrehachse (A) zum Verriegelungsabschnitt (25) erstreckenden Hebelarm aufweisen, wobei sich der jeweilige Hebelarm in der Freigabeposition über das 0,1- bis 0,4-fache eines inneren Umfangs der Okulartrichteraufnahme (21) erstreckt.

14. Kupplungsvorrichtung (15) nach einem der vorhergehenden Ansprüche, wobei die zu jedem der Verriegelungselemente (23) zugehörige Verriegelungsdrehachse (A) jeweils in Tangentialrichtung zur optischen Achse (L) gekippt verläuft.

15. Kupplungsvorrichtung (15) nach einem der vorhergehenden Ansprüche, wobei ein Gemeinlot zwischen der optischen Achse (L) und der Verriegelungsdrehachse (A) zwischen der proximalen Seite (17) der Kupplungsvorrichtung (15) und der distalen Seite (19) der Kopplungsvorrichtung (15) liegt.
